# EUROPEAN PATENT APPLICATION

(11) **EP 1 505 157 A1**
(43) Date of publication of application: **09.02.2005**
(21) Application number: 03017496.5
(22) Date of filing: 02.08.2003
(51) Int. Cl.: C12Q 1/68, C12N 15/74

(54) **Screening assay for anti-bacterial compounds**

(71) Applicant: Bayer HealthCare AG, 51368 Leverkusen (DE)
(72) Inventor: Ehlert, Kerstin, Dr., 42115 Wuppertal (DE); Binas, Annegret, 42553 Velbert (DE)

(57) **Abstract**

The invention relates to methods of screening for compounds having anti-microbial efficacy. More specifically the invention relates to transcription/translation assays for the identification of compounds that exhibit inhibitory effects on bacterial growth.

SEQUENCE LISTING

<110>  Bayer AG
<120>  Screening assay for anti-bacterial compounds
<130>  LeA36810-EP
<160>  6

<170>  PatentIn version 3.1

<210>  1
<211>  26
<212>  DNA
<213>  Artificial sequence
<220>
<223>  Primer T7FF1
<400>  1

gcgcggatcc aaaggaaaat aggagg                                          26


<210>  2
<211>  30
<212>  DNA
<213>  Artificial sequence
<220>
<223>  Primer T7FF2
<400>  2

atcctgaaac tgactgaact aattgagtcg                                      30


<210>  3
<211>  34
<212>  DNA
<213>  Artificial sequence
<220>
<223>  Primer Xyl1
<400>  3

gcgcattaag ctttttctca aggcagtcca attc                                 34


<210>  4
<211>  96
<212>  DNA
<213>  Artificial sequence
<220>
<223>  Primer Xyl2
<400>  4

gcgctctaga ggatagaatg gcgccgggcc tttctttatg tttttggcgt cttccataat     60
attcctccta cattttagtt ggttaattta ataaag                                96


<210>  5
<211>  96
<212>  DNA
<213>  Artificial sequence
<220>
<223>  Fig. 1 A
<400>  5

gcgaaattaa tacgactcac tatagggaga ccacaacggt ttccctctag gatccaaagg 60
aaaataggag gtttatatgg aagacgccaa aaacat 96

<210> 6
<211> 104
<212> DNA
<213> Artificial sequence
<220>
<223> Fig. 1 B
<400> 6

tttacaaaaa atgaacaatg tgctatatta cctctaaagt tagtttgttt attaaattaa 60
ccaactaaaa tgtaggagga atattatgga agacgccaaa aaca 104

## Description

The invention relates to methods of screening for compounds having anti-microbial . efficacy. More specifically the invention relates to transcription/translation assays for the identification of compounds that exhibit inhibitory effects on bacterial growth.

### Background

The growing incidence of infections caused by Gram-positive pathogens resistant against multiple antibiotics became a major and rapidly growing clinical problem [1]. One approach to address this problem is the identification of novel antibacterial substance classes showing efficient killing of resistant bacteria by a novel mode of action.

Prokaryotic transcription and translation are essential processes in bacterial growth, which can be efficiently inhibited by marketed antibiotics. Rifampicin acts as an inhibitor of bacterial RNA polymerase, thereby interfering with bacterial transcription. Macrolides, aminoglycosides, tetracyclines and oxazolidinones act on bacterial translation. Unfortunately, upcoming resistance mechanisms particularly in Gram-positive pathogens, e. g. *staphylococci, pneumococci* and *enterocococci,* against these and other antibiotics reduced or abolished the effectiveness of these drugs in antibacterial therapy.

Nevertheless, bacterial transcription and translation processes are regarded as an useful target area providing the potential to identify novel inhibitory compounds.

Bacterial *in vitro* coupled transcription/translation (T/T) assays for *E. coli* are known to the person skilled in the art [7, 8] and a T/T assay is commercially available from Promega (Madison, Wisconsin, USA) for this organism. Other assays for Gram-positive bacteria, which are known to the person skilled in the art, are either radioactive assays [9] or are applicable only for *S. aureus [2].*

Recently, an *E. coli in vitro* coupled transcription/translation assay [10] was disclosed which was adapted to a high-throughput screening (HTS) format.

Based on the above mentioned state of the art, it is an object of the invention to provide improved methods for screening for anti-bacterial compounds, overcoming the various shortcomings of the present state-of-the-art screening methods of the above.

### Description of the invention

The current invention relates to *in vitro* coupled transcription/translation assays.

One aspect of the current invention relates to a non-radioactive *in vitro* coupled T/T assay using firefly luciferase as a reporter gene and S30 extracts from Gram-positive-pathogens (see Example 2), e. g. *staphylococci, pneumococci* and *enterococci,* which is suitable for the rapid identification of novel transcription/translation inhibitors.

Another aspect of the current invention relates to the construction of the plasmid pT7-FF, which enables a non-radioactive *in vitro* coupled transcription/translation assay suitable for all important Gram-positive pathogens including *staphylococci, pneumococci* and *enterococci.*

Still another aspect of the invention relates to an assay that employs S30 extracts from Gram-positive bacteria and/or one of two reporter plasmids, referred to as pT7-FF and pXyl-FF.

The broad applicability of the assays of the invention for various bacterial species due to, e.g., the use of the viral T7 promoter preferably in combination with a Gram-positive ribosome binding site, represents one major contribution of the current invention to the state of the art.

An advantage over the commercially available *E. coli* (T/T) assay mentioned in the preceeding section is the possibility to characterize mutants resistant to inhibitors of bacterial transcription or translation in different Gram-positive species, i.e., it can be investigated whether resistance of a Gram-positive strain is due to target mutations (which should lead to increased IC₅₀ values) or to other mechanism, e.g., reduced uptake of the compound by the bacterial cell or enhanced efflux from the bacterial cell.

The described assays facilitate the identification of novel potential inhibitors of bacterial translation in medically relevant Gram-positive pathogens.

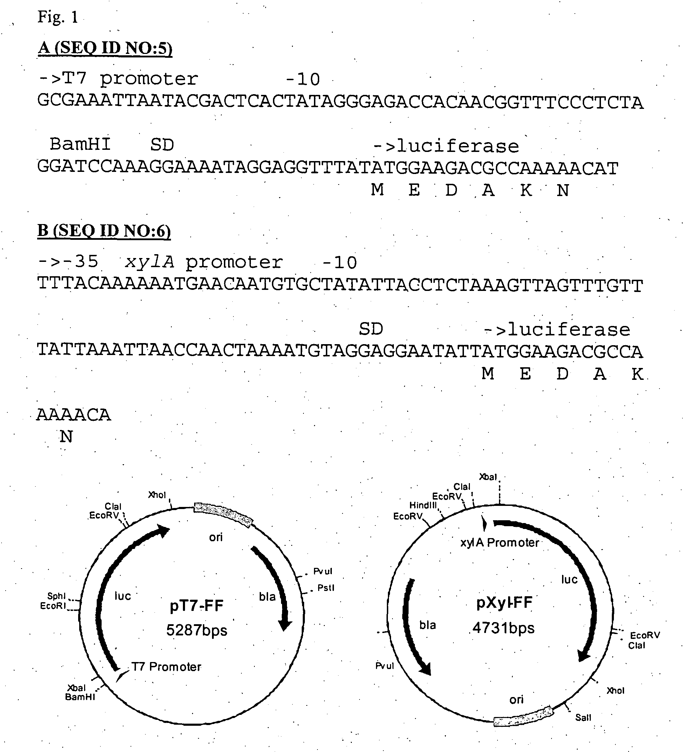

Two reporter plasmids, pT7-FF and pXyl-FF were constructed using S30 extracts from Gram-positive bacteria, for use in the *in vitro* coupled transcription/translation assay. Both plasmids contain the eukaryotic firefly luciferase gene as reporter gene enabling a bioluminescence read-out, which can be easily measured. Plasmid pT7-FF contains the bacteriophage T7 promoter and the SD sequence from the S. *aureus* capAl promoter [2], whereas plasmid pXyl-FF contains the SD and promoter sequences of the S. xylosus xylA promoter [3] (Fig. 1): Both plasmids, pT7-FF and pXyl-FF, can be easily propagated in E. *coli.* The xylA promoter was choosen . because of its strong expression in *staphylococci* [3]. The T7 promoter is also known as a strongly transcribed promoter and an *in vitro* coupled transcription/translation assay using S30 extracts from *E. coli* supplemented with T7 RNA polymerase is commercially available from Promega Corporation. Moreover, a radioactive *in vitro* transcription/translation assay driven by the T7 promoter was described for S. *carnosus* [4].

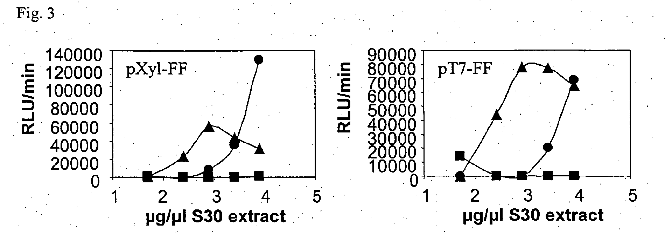

As can be seen from Fig. 3 the amount of bioluminescence did not correlate with the amount of S30 extract used in *the in vitro* coupled T/T assay. Whereas S30 extracts from *S. aureus* caused increasing luminescent signals when used at higher concentration, an "optimal" extract concentration was determined for *S. pneumoniae* and *E. faecalis* (Fig. 3). Based on these results it can be important that each new preparation of a Gram-positive S30 extract or plasmid DNA for an *in vitro* coupled transcription/translation assay is titrated to determine optimal concentrations for the assay, which might even be necessary when equal protein and DNA concentrations for the different preparations were determined. The reason for this phenomenon is not clear, but it was also observed by other authors [2]. It might be that slight variations in the transcriptional and translational enzymatic activities in different S30 extract preparations are the reason for this phenomenon. For an *E. coli in vitro* T/T assay it was assumed that the proper cultivation conditions as well as the preincubation step are essential for the successful isolation of fully active S30 extracts [5]. Additionally, it was assumed that differences in translational activity could be due to differences in post-translational protein modifications [5].

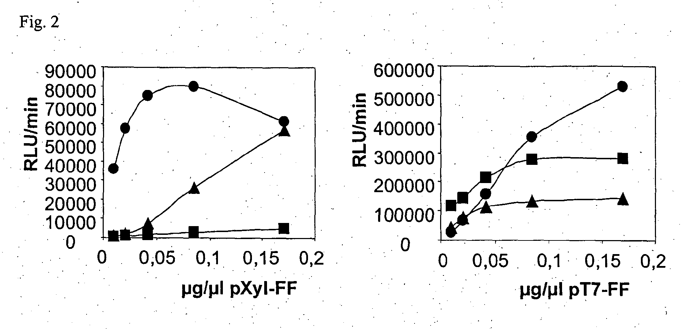

The production of *in vitro* synthesized luciferase is dependent on the concentration of template DNA, although a plateau of bioluminescence is reached at a certain amount of DNA template, which cannot be further increased (Fig. 2). Whereas pXyl-FF is suitable for *in vitro* coupled transcription/translation assays with S30 extracts isolated from S. *aureus* and *E. faecalis;* it results in only low bioluminescence when used as a transcriptional template with S30 extracts from *S*. *pneumoniae* (Fig. 2a). Furthermore, high template concentrations of pXy1-FF are required for optimal translation of luciferase by *E. faecalis* S30 extracts (Fig. 2a). Transcription of the *xylA* promoter seems to be lower by the enterococcal RNA polymerase compared to the staphylococcal RNA polymerase and might be nearly inefficient with the pneumococcal RNA polymerase. This assumption is further supported by the fact that pT7-FF used as a transcriptional template supplied with T7 polymerase results in a high production of luciferase with all three Gram-positive S30 extracts (Fig. 2b).

"% identity" of a first sequence towards a second sequence, within the meaning of the invention, means the % identity which is calculated as follows: First the optimal global alignment between the two sequences is determined with the CLUSTAL W algorithm [11], Version 1.8, applying the following command line syntax: ./clustalw -infile=./infile.txt -output= -outorder=aligned -pwmatrix=gonnet -pwdnamatrix=clustalw -pwgapopen=10.0 -pwgapext=0.1 -matrix=gonnet -gapopen=10.0 -gapext=0.05 -gapdist=8 -hgapresidues=GPSNDQERK -maxdiv=40. Implementations of the CLUSTAL W algorithm are readily available at numerous sites on the internet, including, e.g., http://www.ebi.ac.uk. Thereafter, the number of matches in the alignment is determined by counting the number of identical nucleotides (or amino acid residues) in aligned positions. Finally, the total number of matches is divided by the total number of nucleotides (or amino acid residues) of the longer of the two sequences; and multiplied by 100 to yield the % identity of the first sequence towards the second sequence.

A "promoter", within the meaning of the invention, is a DNA region, usually upstream of a gene or operon, which binds to RNA polymerase and/or directs RNA polymerase to the correct transcriptional start site and thereby permits the initiation of transcription. Other factors, affecting transcription efficacy can also bind to promotor regions or DNA regions close to the promotor regions.

A "T7 promoter", within the meaning of the invention, is a promoter of the T7 bacteriophage.

A "ribosome binding site", within the meaning of the invention, is a DNA region to coding for an RNA region to which a ribosome binds for initiation of translation.

"Gram-positive", within the meaning of the invention, shall be understood as being a phenotypic trait of bacteria, which becomes manifest in that bacteria are stainable by the well known Gram staining procedure.

A "reporter gene", within the meaning of the invention, shall be understood as being a DNA region coding for a protein which, when expressed, is readily detectable, traceable, and/or measurable by methods already known in the art or by methods yet to be conceived.

"Operably linked", with respect to a gene of interest and a regulatory sequence, within the meaning of the invention, shall be understood as the gene of interest and the regulatory sequence being situated on a nucleic acid molecule, such that the regulatory sequence can exhert its regulatory effect on the expression of the gene of interest.

A "luciferase", within the meaning of the invention, shall be understood as being any protein having luminescent properties. A preferred luciferase is the firefly luciferase, more preferred the Photinus-luciferin 4-monooxygenase (ATP-hydrolyzing, EC1.13.12.7) *of Photinus pyralis.*

A "selection marker", within the meaning of the invention, shall be understood as being a DNA region that codes for a protein, conferring to an organism a phenotype which can be readily selected for. Preferred selection markers are genes conferring resistance to antibiotics or genes overcoming an auxotrophy of the organism. Other preferred selection markers change the physical appearance of the organism so that organisms expressing the selection marker can easily be distinguished from those that do not express the selection marker.

. An "origin of replication" with respect to a plasmid, within the meaning of the invention, shall be understood as being a DNA region which serves as a starting point for the replication of said plasmid in a host cell.

"Anti-microbial activity" with respect to a chemical or biological compound, within the meaning of the invention, shall be understood as being, e.g., the inhibiting effect of said compound towards microbial growth or proliferation or the mortifying effect of said compound with respect to a microbial species. Preferred compounds having anti-microbial activity have been collectively referred to as "antibiotics". ..

A "cell extract", within the meaning of the invention, shall be understood as being any fraction of the cell's constituents which can be obtained from a sample of said cells, with or without the step of disrupting the cells. Preferred cell extracts are obtained from a cell debris after disruption of the cells. Other preferred cell extracts can be obtained by extraction procedures using hydrophobic or hydrophilic extraction media.

"S30 extracts", within the meaning of the invention, shall have the ordinariy meaning of this term, known to the person skilled in the art. A preferred S30 extract is an extract prepared according to example 2 of the current application.

A "high-throughput" format, with respect to an assay or a screening method, within the meaning of the invention, shall be understood as being an assay format; suitable for carrying out large numbers of assays in parallel and/or in sequence. Preferred high-throughput assays are performed on multiwell solid supports, such as 96-, 384-, 1536-plates. Other preferred high-throughput assays are performed on solid supports applying nano-technology.

The invention relates to:
1. DNA material comprising either a T7 promoter or the xylA promoter, and a ribosome binding site from a Gram-positive bacterium, and a reporter gene, which is operably linked to the promoter. In a preferred embodiment, said DNA material is a plasmid.
2. DNA material of count 1, wherein the reporter gene is a luciferase. In a preferred embodiment of the invention, the luciferase is a firefly luciferase.
3. DNA material of any of counts 1 or 2, wherein the plasmid additionally comprises a selection marker and/or an origin of replication. Preferred selection markers are genes conferring resistance to antibiotics, such as e.g., canamycin or ampicillin.
4. DNA material of any of counts 1 to 3, wherein the DNA material comprises a sequence selected from the group comprising
   (i) the sequence of SEQ ID NO:5;
   (ii) a sequence at least 90% identical to SEQ ID NO:5;
   (iii) the sequence of SEQ ID NO:6; and
   (iv) a sequence at least 90% identical to SEQ ID NO:6.
   In other embodiments of the invention, the sequence identity under (ii) and (iv) above is at least 80%, 95%, 98%, or 99%.
5. A method to determine whether a test substance has anti-microbial activity against Gram-positive bacteria, comprising the steps of
   (i) incubating the test substance with bacterial cell extract of a Gram-positive bacterium and the DNA material of any of counts 1 to 4; and
   (ii) detecting a signal resulting from the expression of said reporter gene.
   In a preferred embodiment of the invention, T7 RNA polymerase is added to the incubation mixture of step (i), and the applied DNA material comprises the T7 promoter. In another preferred embodiment, the signal detected is luminescence of the expression product of the reporter gene.
6. The method of count 5 wherein the Gram-positive bacterium is *Staphylococcus, Pneumococcus, Enterococcus.*
7. The method of count 5 or 6, wherein said bacterial cell extract is a bacterial S30 cell extract.
8. The method of count 6 or 7, wherein said incubation is on a multi-well plate, suitable for use in a plate reader.

### Description of figures

Figure 1
   shows promoter sequences and plasmid maps of the reporter plasmids pT7-FF (A) and pXyl-FF (B). In promoter sequences the promoter region, relevant restrictions sites, Shine-Dalgarno (SD) sequences and N-terminal regions of the firefly luciferase are shown.
Figure 2
   shows a Gram-positive *in vitro* coupled T/T assay using increasing concentrations of plasmid DNA template pXyl-FF (A) or pT7-FF (B). S30 extracts from *S*. *aureus 133* (●)*, S*. *pneumoniae* 1707/4 (■) or *E. faecalis* 27263 (**▲**) were used. Assay conditions were as described in Example 3.
Figure 3
   shows a Gram-positive *in* vitro-coupled-T/-T-assay using increasing concentrations of S30 extracts from S. *aureus* 133 (●)*, S. pneumoniae* 1707/4 (■) or *E. faecalis* 27263 (A). Assay conditions were as described in Example 3.
Figure 4:
   shows dose-response inhibition profiles of various known antibiotics determined by the *in vitro* coupled T/T assay. Plasmid pT7-FF (A) or pXyl-FF (B) were used as a DNA templates and tested with S30 extracts from *S. aureus* 133 (A) or *E. faecalis* 27263 (B).

### Examples

### Example 1: Construction of the Gram-positive luciferase reporter plasmids

Plasmid pT7-FF was constructed using pET-23(+) (Novagen) providing a T7 promoter sequence, which was digested with BamHI and XhoI. Plasmid pSAluc was constructed as described (2) and used as a template to amplify the firefly luciferase gene containing the Gram-positive SD sequence from the *S. aureus* cap1A promoter. Using the primer pair T7FF 5'-GCGCGGATCCAAAGGAAAATAGGAGG-3' (SEQ ID NO:1) and T7FF2 5'-ATCCTGAAACTGACTGAACTAATTGAGTCG-3'(SEQ ID NO:2) a BamHI restriction site was introduced at the 5'-region of the firefly luciferase gene. The resulting 1.6 kb PCR product was digested with BamHI and XhoI, the latter restriction site is present downstream from the firefly luciferase gene (2). The digested PCR product was ligated into the digested plasmid pET-23(+) and transformed into *E. coli* JM 110.

Plasmid pBESTIuc (Prorriega) providing the firefly luciferase gene was used to construct plasmid pXyl-FF. pBESTIuc was digested with HindIII and XbaI to remove the *E. coli* promoter and SD sequence. The xylA promoter from S. xylosus C2a (DSM 20267) was PCR amplified using the primers Xyll 5'-GCGCATTAAGCTTTTTCTCAAGGCAGTCCAATTC-3'(SEQ ID NO:3) introducing a HindIII site and Xyl2 5'-GCGCTCTAGAGGATAGAATGGCGCCGGGCCTTTCTTTATGTTTTTGGCGT CTTCCATAATATTCCTCCTACATTTTAGTTGGTTAATTTAATAAAG-3'(SEQ ID NO:4). Primer Xyl2 encodes a XbaI site, the SD sequence from the S. xylosus xylA promoter and the 5'-end from the firefly luciferase gene. The resulting 0.4 kb PCR product was digested with HindIII and XbaI and ligated into the predigested plasmid pBESTIuc prior to transformation into *E. coli* JM 110.

### Example 2: Preparation of S30 extracts.

A procedure similar to the preparation of S30 extracts described by Murray et. al. (2) was used for *S. aureus.* Brain heart infusion (BHI) medium (6 1) was inoculated with 250 ml overnight culture of S. *aureus* 133 grown in BHI at 37°C with agitation (170 rpm). Cells were cultured at 37°C until an OD₅₉₅ₙₘ of 2.5 was reached prior to centrifugation at 6000 x g for 15 min at 4°C. Pellets were washed once with 500 ml cold S30-buffer A (10 mM Tris-acetate pH 8.0, 14 mM Mg-acetate, 1 mM DTT, 1 M KCl) and once with 250 ml of cold S30-buffer A containing 50 mM KCl. Pellets were frozen for 1 h at -20°C or overnight at -70°C, thawed on ice and resuspended to a final volume of 99 ml buffer B (10 mM Tris-acetate pH 8.0, 20 mM Mg-acetate, 1 mM DTT, 50 mM KCl). A 1.5 ml aliquote of lysostaphin (0.8 mg/ml) in buffer B was added to each of three centrifuge tubes and 33 ml of the cell suspension were added. Samples were incubated at 37°C for 1 h with gentle agitation prior to the addition of 150 µl 0.5 M DTT. Lysed cells were centrifuged for 30 min at 30,000 x g at 4°C and the resulting pellet was washed once with 33 ml S30-buffer B. The pooled Supernatants were recentrifuged at 30,000 x g for 30 min at 4°C. 100 ml of the supernatant were incubated with 25 ml preincubation buffer (670 mM Tris-acetate pH 8.0, 20 mM Mg-acetate, 7 mM Na3-PEP, 7 mM DTT, 5.5 mM ATP, 70 µM amino acids complete [Promega], 75 µg pyruvate kinase [Sigma]/ml) for 30 min at 37°C to translate mRNAs. The preincubated supernatants were dialysed at 4°C against 21 of 10 mM Tris-acetate pH 8.0, 14 mM Mg-acetate, 1 mM DTT, 60 mM KCl with one buffer change using a Spectra-Por dialysis bag with a molecular weight cutoff of 3,500. The dialysate was gently concentrated by covering the dialysis bag with polyethylene glycol 8,000 powder (Sigma) at 4°C to a protein concentration of ~ 10 mg/ml. The resulting S30 extracts were flash frozen and stored in aliquots at -70°C.

*S. pneumoniae* 1707/4 was grown overnight at 37°C on agar plates containing 5 % sheep blood under microaerophilic conditions. Cells were collected and inoculated into 4.8 l of BHI medium containing 5 % bovine serum prior to cultivation at 37°C under microaerophilic conditions for 5 h. S30 extracts were prepared as described for *S. aureus* 133 with some modifications. Cells were harvested and washed by centrifugation at 20,000 x g for 15 min. The resulting cell pellet was resuspended in 20 ml buffer B, 3 mg lysozyme were added, incubated for 40 min at 37°C prior to the addition of 75 µl 0.5 M DTT. The S30 sediment was washed with 8 ml buffer B and 7 ml preincubation buffer were added to the pooled supernatants.

*E. faecalis* 26273 was grown overnight in 250 ml BHI medium containing 2 % bovine serum at 37°C under microaerophilic conditions. The overnight culture was used to inoculate 4.8 l BHI medium, cells were incubated at 37°C under microaerophilic conditions with agitation (170 rpm) until an OD600nm of ~1.6 was reached. Preparation of S30 extracts was done as described for,*S. aureus* with the following exceptions. Cells were harvested and washed at 7000xg at 4°C for 12 min and resuspended in 100 ml S30-buffer B. For cell lysis 15 mg lysozyme resuspended in 1 ml S30-buffer B were added and incubated for 2 h at 37°C with agitation. To complete cell lysis extracts were passed once through a French Press at 600 psi.

### Example 3: Gram-positive in vitro coupled transcription/translation assays.

The final assay volume was 59 µl. Inhibitor (3 µl dissolved in 10% DMSO), plasmid DNA (10 µl of optimal concentration in water pH 8.0) and 46 µl S30 extract [optimal concentration in 23 µl premixed with 23 µl incubation buffer (0.5 M potassium acetate, 87.5 mM Tris/acetate pH 8.0, 67.5 mM ammonium acetate, 50 µg/ml folinic acid, 5 mM DTT, 87.5 mg/ml polyethylene glycol 8000, 5 mM ATP; 1.25 mM of each CTP, GTP and UTP; 0.02 mM amino acids, 50 mM phosphoenolpyruvate trisodium salt, 2.5 mM cyclic AMP, 250 µg of each *E. coli* tRNA/ml) were added and incubated at 30°C for 1 h. When using pT7-FF as a template 0.05 µl or 0.1 µl (50 U/µl) T7-RNA-Polymerase (Gibco BRL) were added to the S30 extracts used in the assay. Newly synthesized luciferase was detected by measuring bioluminescence for 60 s in a luminometer after the addition of 50 µl substrate buffer containing luciferine (20 mM Tricine/Cl pH 7.8, 2.67 mM MgSO₄, 0.1 mM EDTA, 33.3 mM DTT, 0.27 mM Coenzyme A, 0.47 mM luciferine, 0.53 mM ATP).

### Example 4: Determination of the minimum inhibitory concentration (MIC)

MIC determinations were performed using the micro broth dilution method with an inoculum of 5 x 10⁵ cfu/ml in BHI medium. Growth was read after 24 h of incubation at 37°C. Test compounds were dissolved in dimethyl sulfoxide and diluted to a concentration not higher than 2.5 % dimethyl sulfoxide. For *S. pneumoniae* 1707/4 and *E. faecalis* 27263 10 % bovine serum were added and incubated under microaerophilic conditions.

### Example 5: Validation of the assay

To validate the *in vitro* coupled T/T assay for screening of potential inhibitors of transcription and translation several antibiotics of known mode of action were tested. All known inhibitors of protein biosynthesis showed IC₅₀ values in the low µM or nM range when tested with different templates and S30 extracts derived from all three Gram-positives used in this work (Table 1). In contrast, inhibitors of cell wall biosynthesis, e. g. ampicillin and vancomycin, as well as the DNA gyrase inhibitor moxifloxacin showed no or only weak inhibition at high concentrations when tested in the *in vitro* coupled T/T assay (Table 1). The results clearly show the specificity of the developed assay for the detection of protein biosynthesis inhibitory compounds. It can also be seen that the usage of different templates resulted in similar IC₅₀ values for a given bacterial species (Table 1). The IC₅₀ values differed between the tested Gram-positive species in some cases: Erythromycin showed a lower IC₅₀ when tested with the pneumococcal S30 extract compared to *S. aureus* 133 or *E. faecalis* 27263 (Table 1). Synercid showed an increased IC₅₀ value for *E. faecalis* 27263 in comparison to *S. aureus* 133 or *pneumoniae* 1707/4 (Table 1), which correlates with the spectrum gap of this drug with regard to *E. faecalis* (6). 'Streptomycin was also less effective when tested with the enterococcal S30 extract (Table 1).

### Example 6: Validation with Rifampicin

Rifampicin, known as an inhibitor of bacterial RNA polymerases, showed low IC₅₀ values in the nM range when transcription of luciferase was driven by the bacterial xylA promoter encoded by pXyl-FF (Table 1). Since viral transcription is not inhibited by Rifampicin, no inhibition of luciferase production was observed with pT7-FF as DNA template encoding the bacteriophage T7 promoter. Therefore, the mode of action of potential inhibitory compounds can be distinguished with respect to transcription or translation when tested with both DNA templates.

### Example 7: Comparison of IC₅₀ with MIC

Regarding the correlation between the IC₅₀ values and their corresponding MICs it can be seen that for most inhibitors the IC₅₀ values are lower or in the range of the MIC values. For tetracyclin a lower MIC compared to the IC₅₀ value was found for *S. aureus* (Table 1). The reason for this is not clear, but might be due to the fact that enzymatic extracts are used in the *in vitro* coupled T/T assay rather than purified enzymes. The different protein translation inhibitors act on different sites during protein translation and the rate limiting step in *the in vitro* T/T assay is unclear.

In general the IC₅₀ values for known antibiotics determined with the *described in vitro* coupled T/T assay were highly reproducible and were comparable to recently published data for *S. aureus* in a similar assay system (2). For *E. faecalis* 27263 increased or no MIC values were determined for most protein translation inhibitors despite the detection of low IC₅₀ values (Table1). It might be that this strain is resistent to these antibiotics due to reduced uptake or enhanced efflux mechanisms.

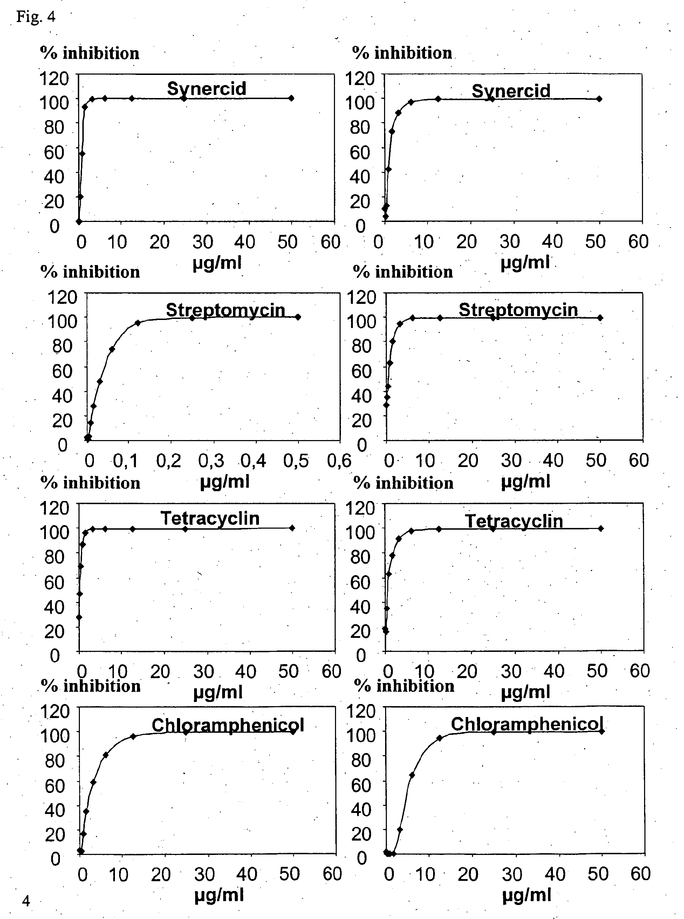

The dose-response inhibition profiles for Synercid, Streptomycin, Tetracyclin and Chloramphenicol are shown in Fig. 4. The shape of the inhibition profiles is more or less similar for these compounds, in case of Streptomycin the lower IC₅₀ value for *S. aureus* -133 compared to E. *faecalis* 27263 can be seen.

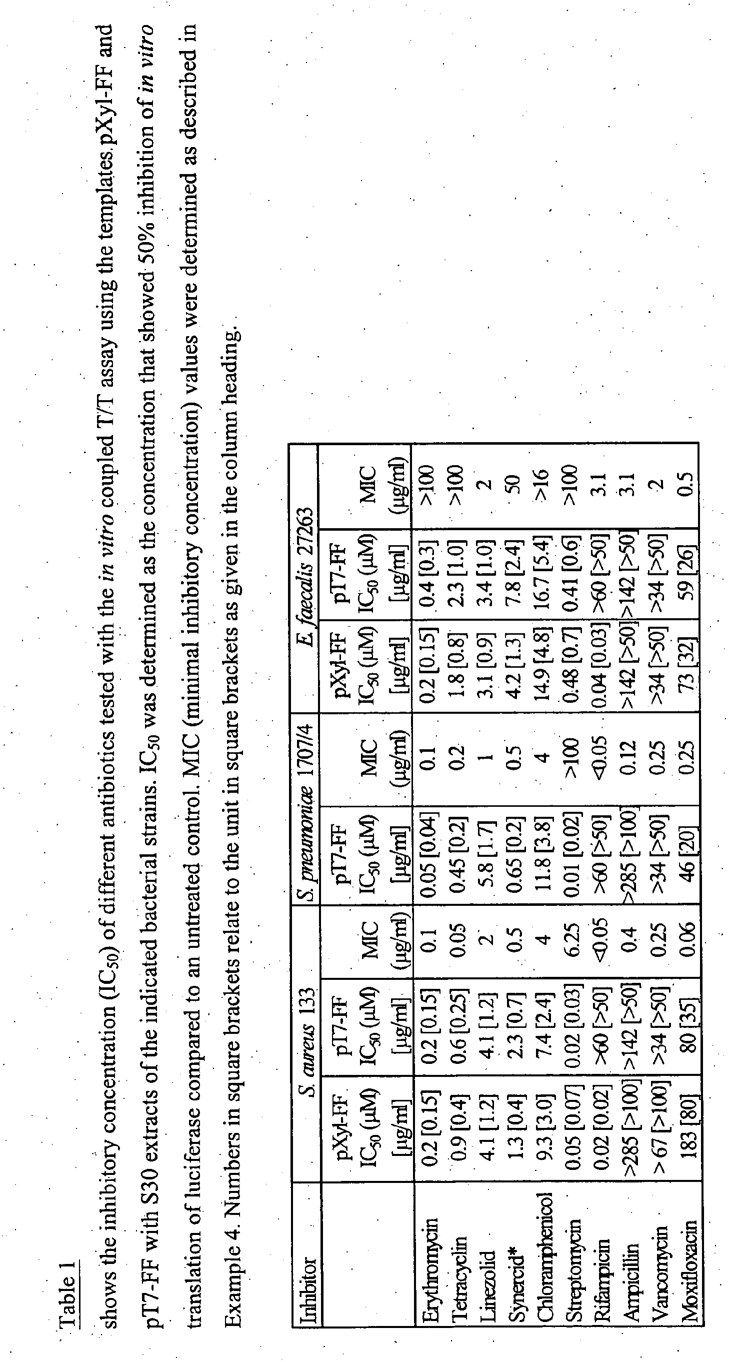

### References

The following references were considered to be relevant to various aspects of the invention:
- [1]: Tomasz, A. 1994. Multiple-antibiotic-resistant pathogenic bacteria. N. Engl. J. Med. 330:1247-1251.
- [2]: Murray RW, Melchior EP, Hagadom JC, Marotti KR. 2001. Staphylococcus aureus cell extract transcription-translation assay: firefly luciferase reporter system for evaluating protein translation inhibitors. Antimicrob. Agents Chemother. 45:1900-1904.
- [3]: Sizemore C, Wieland B, Götz F, Hillen W. 1992. Regulation of Staphylococcus xylosus xylose utilization genes at the molecular level. J. Bacteriol. 174:3042-3048.
- [4]: Schimz KL, Decker G, Frings E, Meens J, Klein M, Muller M. 1995. A cell-free protein translocation system prepared entirely from a gram-positive organism. FEBS Lett. 362:29-33.
- [5]: Schindler PT, Baumann S, Reuss M, Siemann M. 2000. *In vitro* coupled transcription translation: effects of modification in lysate preparation on protein composition and biosynthesis activity. Electrophoresis 21:2606-2609.
- [6]: Collins LA, Malanoski GJ, Eliopoulos GM, Wennersten CB, Ferraro MJ, Moellering RC Jr. 1993. *In vitro* activity of RP59500, an injectable streptogramin antibiotic, against vancomycin-resistant gram-positive organisms. Antimicrob. Agents Chemother. 37:598-601.
- [7]: Zubay G. 1973. *In vitro* synthesis of protein in microbial systems. Annu. Rev. Genet. 7:267-287.
- [8]: Pratt, JM. 1984. Coupled transcription-translation in prokaryotic cell-free systems. In: Hames, B., Higgins, S. (Eds.) Transcription and translation. A practical approach, IRL Press, Oxford, England. 179-209.
- [9]: Mahmood R, Compagnone-Post P, Khan SA. 1991. An *in vitro* coupled transcription- translation system from Staphylococcus aureus. Gene 106:29-34.
- [10]: Kariv I, Cao H, Marvil PD, Bobkova EV, Bukhtiyarov YE, Yan YP, Patel U, Coudurier L, Chung TD, Oldenburg KR. 2001. Identification of inhibitors of bacterial transcription/translation machinery utilizing a miniaturized 1536-well format screen. J. Biomol. Screen. 6:233-243.
- [11]: Thomson JD, Higgins DG, gibson TJ. 1994. ClustalW: Improving the sensitivity of progressive multiple sequence alignment through sequence weighting, positions-specific gap penalties and weight matrix choice. Nuleic Acids Res., 22: 4673-4680.

## Claims

1. DNA material comprising either a T7 promoter or the xylA promoter, and a ribosome binding site from a Gram-positive bacterium, and a reporter gene, which is operably linked to the promoter.

2. DNA material of claim 1, wherein the reporter gene is a luciferase.

3. DNA material of any of claims 1 or 2, wherein the plasmid additionally comprises a selection marker and/or an origin, of replication.

4. DNA material of any of claims 1 to 3, wherein the DNA material comprises a sequence selected from the group comprising
(i) the sequence of SEQ ID NO: 5;
(ii) a sequence at least 90% identical to SEQ ID NO:5;
(iii) the sequence of SEQ ID NO:6; and
, (iv) a sequence at least 90% identical to SEQ IID NO:6.

5. A method to determine whether a test substance has anti-microbial activity against Gram-positive bacteria, comprising the steps of
(i) incubating the test substance with bacterial cell extract of a Gram-positive bacterium and the DNA material of any of claims 1 to 4; and
(ii) detecting a signal resulting from the expression of said reporter gene.

6. The method of claim 5 wherein the Gram-positive bacterium is *Staphylococcus, Pneumococcus* or *Enterococcus.*

7. The method of claim 5 or 6, wherein said bacterial cell extract is a bacterial S30 cell extract.

8. The method of claim 6 or 7, wherein said incubation is on a multi-well plate, suitable for use in a plate reader.
